# EUROPEAN PATENT APPLICATION

(11) **EP 1 219 172 A1**
(43) Date of publication of application: **03.07.2002**
(21) Application number: 01310457.5
(22) Date of filing: 14.12.2001
(51) Int. Cl.: A01N 37/34, A01N 25/02

(54) **Halocyanoacetamide antimicrobial compositions**

(30) Priority: 28.12.2000 US 258480 P
(71) Applicant: ROHM AND HAAS COMPANY, Philadelphia, Pennsylvania 19106-2399 (US)
(72) Inventor: Gironda, Kevin F., Telford, Pennsylvania 18976 (US); Mattox, John Robert, Perkasie, Pennsylvania 18944 (US); Nichols, Richard W., Washington Crossing, Pennsylvania 18977 (US); Pressley, Andre Elvis, Philadelphia, Pennsylvania 19150 (US); Redlich, George Harvey, Norristown, Pennsylvania 19403 (US); Yu, Bing, Ambler, Pennsylvania 19002 (US)
(74) Representative: Kent, Venetia Katherine

(57) **Abstract**

A stabilized halocyanoacetamide composition in the form of a solution or emulsifiable concentrate using selected ester solvents is disclosed. Preferred ester solvents include glyceryl triacetate, ethyl acetate and diethyl phthalate. Particularly preferred are stabilized compositions containing 2,2-dibromo-3-nitrilopropionamide, glyceryl triacetate and optional non-ionic surfactants or additional antimicrobial compounds, particularly 3-isothiazolone compounds.

## Description

### BACKGROUND

This invention relates to stable antimicrobial compositions comprising halocyanoacetamides and selected ester solvents. In particular the invention involves stable compositions of 2,2-dibromo-3-nitrilopronioamde, further comprising selected 3-isothiazolone compounds.

2,2-Dibromo-3-nitrilopropionamide (DBNPA) is an antimicrobial compound used commercially for inhibiting the growth of microbial organisms in industrial applications. DBNPA is typically sold as a 20% active ingredient solution in a mixture of glycol and water.

Japanese Patent Application J 62-048603A discloses synergistic industrial antimicrobial compositions containing at least two or more of 2,2-dibromo-3-nitrilopropionamide, 4,5-dichloro-1,2-dithiol-3-one and hexachloromethylsulfone as its effective components. The reference also discloses a wide range of solvents, including alcohols, ketones, ethers, aliphatic hydrocarbons, aromatic hydrocarbons, halogenated hydrocarbons, amides, esters and nitriles as carriers for the compositions.

Although glycol and water solutions of DBNPA are sold commercially, the active ingredient is unstable in these solutions leading to discoloration of the solution. The problem addressed by the present invention is to provide stable compositions of halocyanoacetamide compounds that offer efficient use under commercial conditions.

### SUMMARY OF INVENTION

The present invention provides a composition comprising (a) a halocyanoacetamide compound of formula **I**: 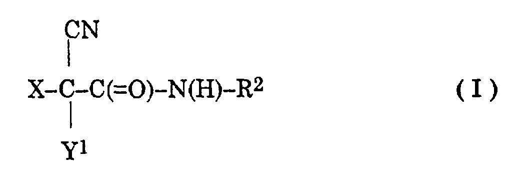 wherein X is hydrogen or halogen atom, Y¹ is halogen atom and R² is hydrogen or (C₁-C₆)alkyl group; and (b) a solvent comprising a (C₁-C₄)alkyl ester of (C₁-C₃)aliphatic carboxylic acid or (C₇-C₉)aromatic carboxylic acid.

In a preferred embodiment, the present invention provides the aforementioned composition further comprising a 3-isothiazolone compound of formula **II**: 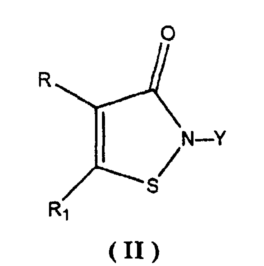 wherein Y is an unsubstituted or substituted (C₁-C₁₈)alkyl group, an unsubstituted or substituted (C₃-C₁₈)alkenyl or alkynyl group, an unsubstituted or substituted (C₅-C₁₂)cycloalkyl group, an unsubstituted or substituted (C₇-C₁₀)aralkyl group, or a substituted or unsubstituted (C₇-C₁₀)aryl group; R and R₁ are independently hydrogen, halogen or (C₁-C₄)alkyl groups; or R and R₁ can be taken together with the C=C double bond of the isothiazolone ring to form an unsubstituted or substituted benzene ring.

In another preferred embodiment, the present invention provides the aforementioned composition wherein the halocyanoacetamide is 2,2-dibromo-3-nitrilopropionamide, the solvent is glyceryl triacetate and the 3-isothiazolone is 4, 5-dichloro-2-n-octyl-3-isothiazolone.

The present invention further provides a method for stabilizing halocyanoacetamide compounds of formula **I** comprising (a) forming a solution by combining the halocyanoacetamide with an ester solvent comprising a (C₁-C₄)alkyl ester of (C₁-C₃)aliphatic carboxylic acid or (C₇-C₉)aromatic carboxylic acid; or (b) forming an emulsifiable concentrate by combining the halocyanoacetamide with the ester solvent and one or more surfactants selected from non-ionic, cationic and anionic surfactant.

The present invention further provides a method for controlling the growth of bacteria, fungi, algae and yeasts comprising introducing to a locus to be protected the aforementioned compositions.

### DETAILED DESCRIPTION

We have discovered that certain selected ester solvents provide stability for halocyanoacetamide compounds when the esters solvents are used to prepare solutions or emulsifiable concentrates of the halocyanoacetamides.

As used herein, the following terms have the designated definitions, unless the context clearly indicates otherwise. The term "microbicide" is considered equivalent to "antimicrobial" as used herein and refers to a compound capable of inhibiting the growth of or controlling the growth of microorganisms at a locus; microbicides include bactericides, fungicides and algaecides. The term "microorganism" includes, for example, fungi, yeast, bacteria and algae. The term "locus" refers to an industrial system or product subject to contamination by microorganisms. All percentages referred to will be expressed in weight percent (%), based on total weight of composition involved, unless specified otherwise. The following abbreviations are used herein: g = grams; ml = milliliter, ppm = parts per million by weight/volume. Unless otherwise specified, ranges listed are to be read as inclusive and combinable and temperatures are in degrees centigrade (°C).

The halocyanoacetamide compound useful in compositions of the present invention is represented by the following Formula (**I**): 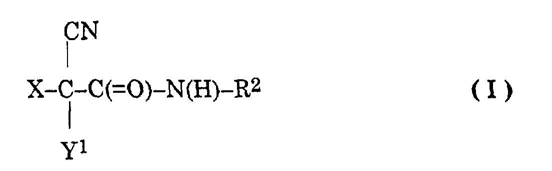 where X is hydrogen or halogen atom, Y¹ is halogen atom and R² is hydrogen or (C₁-C₆)alkyl group. The halogen atom of X and Y¹ is selected independently from fluorine, chlorine, bromine and iodine. X is preferably a hydrogen atom, chlorine or bromine; more preferably X is bromine. Preferably Y is chlorine or bromine; more preferably Y is bromine. Suitable R² alkyl groups include, for example, linear or branched (C₁-C₆)alkyl groups, such as methyl, ethyl, propyl, isopropyl, butyl, isobutyl, sec-butyl, t-butyl, pentyl and hexyl. Preferably R² is a hydrogen atom or (C₁-C₄)alkyl group; more preferably R² is a hydrogen atom or methyl group.

Suitable halocyanoacetamide compounds include, for example, 2,2-dibromo-3-nitrilopropionamide (DBNPA), 2-chloro-3-nitrilopropionamide, 2-bromo-3-nitrilopropionamide, 2,2-dichloro-3-nitrilopropionamide and N-methyl-2,2-dibromo-3-nitrilopropionamide. Preferably the halocyanoacetamide compound is selected from one or more of DBNPA, 2,2-dichloro-3-nitrilo-propionamide and N-methyl-2,2-dibromo-3-nitrilopropionamide; more preferably, the halocyanoacetamide is DBNPA.

The antimicrobial compound in the composition of the present invention may be one compound or may be a mixture of two or more compounds. In the case where the composition contains more than one antimicrobial compound, one of which is halocyanoacetamide, the additional antimicrobial compound is preferably a 3-isothiazolone compound. 3-Isothiazolone compounds useful in the present invention are those represented by Formula (**II**): 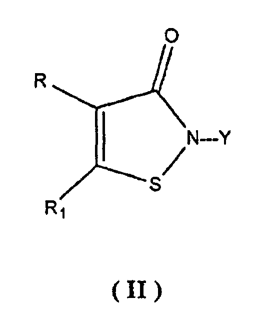 where Y is an unsubstituted or substituted (C₁-C₁₈)alkyl group, an unsubstituted or substituted (C₃-C₁₈)alkenyl or alkynyl group, an unsubstituted or substituted (C₅-C₁₂)cycloalkyl group, an unsubstituted or substituted (C₇-C₁₀)aralkyl group, or a substituted or unsubstituted (C₇-C₁₀)aryl group; R and R₁ are independently selected from hydrogen, halogen and (C₁-C₄)alkyl groups; or R and R₁ can be taken together with the C=C double bond of the isothiazolone ring to form an unsubstituted or substituted benzene ring.

The halogen atom of R and R₁ is independently selected from fluorine, chlorine, bromine and iodine; preferably the halogen atom is chlorine. R and R₁ are preferably hydrogen atom or chlorine, more preferably chlorine.

By a "substituted alkyl group" is meant an alkyl group having one or more of its hydrogens replaced by another substituent group; examples include hydroxyalkyl, haloalkyl and alkylamino. By a "substituted aralkyl group" is meant an aralkyl group having one or more of its hydrogens on either the aryl ring or the alkyl chain replaced by another substituent group; examples include halo, (C₁-C₄)alkyl, halo-(C₁-C₄)alkoxy and (C₁-C₄)alkoxy. By a "substituted aryl group" is meant an aryl group, such as phenyl, naphthyl or pyridyl groups, having one or more of its hydrogens on the aryl ring replaced by another substituent group; examples include halo, nitro, (C₁-C₄)alkyl, halo-(C₁-C₄)alkoxy and (C₁-C₄)alkoxy.

Suitable 3-isothiazolones include, for example, 2-methyl-3-isothiazolone, 5-chloro-2-methyl-3-isothiazolone, 2-n-octyl-3-isothiazolone, 4,5-dichloro-2-n-octyl-3-isothiazolone, 4,5-dichloro-2-cyclohexyl-3-isothiazolone, 4,5-dichloro-2-benzyl-3-isothiazolone, 2-benzyl-3-isothiazolone, 2-cylcohexyl-3-isothiazolone, 2-(4-chlorophenyl)-3-isothiazolone, 2-(4-chlorophenyl)-5-chloro-3-isothiazolone, 2-(4-chlorobenzyl)-3-isothiazolone, 2-(4-chlorobenzyl)-5-chloro-3-isothiazolone, 2-(4-chlorophenethyl)-5-chloro-3-isothiazolone and 2-(4-chlorophenethyl)-3-iso-thiazolone. Preferably, the 3-isothiazolone is selected from one or more of 2-n-octyl-3-isothiazolone, 2-methyl-3-isothiazolone, 5-chloro-2-methyl-3-isothiazolone and 4,5-dichloro-2-n-octyl-3-isothiazolone; more preferably the 3-isothiazolone is 4,5-dichloro-2-n-octyl-3-isothiazolone.

Other suitable microbicidal agents that may be present in the antimicrobial compositions, provided that the physical and chemical stability of the composition is substantially unaffected, include, for example, 3-iodo-2-propynylbutyl-carbamate, 2-bromo-2-nitropropanediol, benzyl alcohol, phenoxyethanol, glutaric dialdehyde, sodium and zinc salts of 2-pyridinethiol-1-oxide, tris(hydroxymethyl)nitromethane, dimethylol-dimethylhydantoin, mono-methylol-dimethylhydantoin, bis-bromoacetoxy butene, 1-bromo-3-chloro-5,5-dimethylhydantoin, sodium dimethyl dithiocarbamate, disodium ethylene bis-dithiocarbamate, potassium N-methyl dithiocarbamate, potassium dimethyl dithiocarbamate, dodecylguanidine hydrochloride, 3,4-dichloro-5-oxo-1,2-dithiol, methylene bis-thiocyanate, pentachlorophenate, N-alkyl dimethyl benzyl ammonium chloride, bis-(trichloromethyl)sulfone, 2-(thiocyano-methythio)-benzothiazol, tetrahydro-3,5-dimethyl-2-H-1,3,5-thiazine-2-thion and benzisothiazolone.

Solvents useful for providing stable solutions of halocyanoacetamides comprise (C₁-C₄)alkyl esters of (C₁-C₃)aliphatic carboxylic acids and (C₇-C₉)aromatic carboxylic acids. Suitable esters include, for example, methyl formate, ethyl formate, propyl formate, butyl formate, methyl acetate, ethyl acetate, propyl acetate, butyl acetate, methyl propionate, ethyl propionate, propyl propionate, butyl propionate, glyceryl triacetate, glyceryl tripropionate, and esters of benzoic acid and phthalic acid, such as dimethyl phthalate, diethyl phthalate, dipropyl phthalate and dibutyl phthalate. Preferably the ester solvent is selected from one or more of glyceryl triacetate, ethyl acetate and diethyl phthalate.

The selected esters act as solvent for halocyanoacetamide and any other antimicrobial compounds that may be present in the composition. The antimicrobial compound exists in the form of a solution dissolved into the ester when the amount of compound in the composition is less than the solubility of the compound in the ester solvent. In the case where the amount of antimicrobial compound being used exceeds its solubility in the solvent, a part of the antimicrobial compound exists in the solid form (a suspension of the antimicrobial compound in a solution of ester solvent). That is, a part of the antimicrobial compound is dissolved in ester solvent and the remainder exists as solid form suspended or dispersed in the ester solution. Preferably, the antimicrobial compound is completely dissolved in the ester solvent to facilitate handling of the composition of the present invention to provide ready diffusion of the composition when applied to various loci.

Since solubility depends on the type of halocyanoacetamide (and any other antimicrobial compounds) and temperature, the amount of antimicrobial compound in the composition depends on the specific compound to be used and the storage temperature for the composition. For example, the solubility of 4,5-dichloro-2-n-octyl-3-isothiazolone in glyceryl triacetate is 20% by weight at 40°C, based on the total amount of 3-isothiazolone and solvent; 8% by weight at 25°C and 8% by weight at 5°C. The solubility of DBNPA (halocyanoacetamide compound) in glyceryl triacetate is 35% by weight at 50°C, based on the total amount of halocyanoacetamide and solvent; 30% by weight at 25°C and 25% by weight at 5°C.

Therefore, for example, when an optional 3-isothiazolone compound, such as 4,5-dichloro-2-n-octyl-3-isothiazolone or 5-chloro-2-methyl-3-isothiazolone, is used, the amount of antimicrobial compound in the composition is generally from 1 to 40%, preferably from 1 to 20% and more preferably from 4 to 8% by weight, based on total weight of the composition. When the halocyanoacetamide compound is DBNPA, the amount of the antimicrobial compound in the composition is generally from 1 to 50%, preferably from 5 to 40% and more preferably from 15 to 25% by weight, based on total weight of the composition.

Optionally, other conventional additives may be present in the antimicrobial compositions of the present invention provided that the physical and chemical stability of the composition is substantially unaffected. For example, surfactants may be present in the composition, including non-ionic, cationic and anionic surfactants. Preferably the surfactants are of the non-ionic type where the composition is substantially free of cationic and anionic surfactants, that is, less than 1%, more preferably less than 0.5% and most preferably less than 0.1% of cationic or anionic surfactant, based on total weight of the composition.

Suitable non-ionic surfactants include, for example, ethoxylated (C₈-C₂₀)alcohols, ethoxylated sorbitan esters, ethoxylated (C₁₀-C₂₀)oils and fats, ethoxylated (C₁₀-C₂₀)fatty acids, ethoxylated (C₈-C₂₀)monoalkyl- or dialkyl-phenols, alkylaryl polyethers, ethoxylated alkanolamides and ethoxylated castor oil.

Suitable surfactants include, for example, polyoxyethylene(20)sorbitan monolaurate (such as Tween™ 20, available from Wako Pure Chemical Co.), modified polyethoxylate straight chain alcohol (such as Triton™ DF-12, available from Union Carbide Co.), alkylaryl polyether (such as, Triton™ CF-10, available from Union Carbide Co.). The surfactant may be used alone or in combination with other surfactants.

The amount of surfactant used in the antimicrobial composition depends on the type of surfactant, type of antimicrobial compound and the locus to which the composition is applied. In general, the amount of the surfactant is from zero to 25%, more preferably from 1 to 15% and most preferably from 2 to 5%, based on the total weight of the composition.

By including a surfactant in the composition, the composition readily forms an emulsion within an aqueous system to which the antimicrobial composition is applied when the halocyanoacetamide compound is dissolved in the ester solvents. When a locus to which a composition of the present invention is applied is an aqueous system, the composition forms an emulsion and the composition can be easily diffused into the locus with enhanced effectiveness of the halocyanoacetamide compound.

Optionally, the composition of the present invention may contain water. For example, when the solvent is substantially glyceryl triacetate, it is permitted to contain small amount of water, typically 10% or less, preferably 5% or less, and more preferably less than 1%, based on total weight of the composition. When forming emulsions from the compositions of the present invention, it is permitted to use larger quantities of water sufficient to form the emulsion. Preferably, the compositions are substantially free of water, that is, less than 10%, more preferably less than 5% and most preferably less than 1%, based on total weight of the composition.

Optionally, other organic solvents may be present in the antimicrobial compositions of the present invention provided that the physical and chemical stability of the composition is substantially unaffected. In general, the amount of optional organic solvent in the composition is 50% by weight or less, and preferably 5% by weight or less. Additional optional conventional additives may be included, for example, coloring agents, pesticides and perfumes.

Typically, compositions of the present invention are those where the halocyanoacetamide comprises 10 to 30%, preferably 15 to 25% and more preferably 20 to 25%; the solvent comprises 40 to 90%, preferably 50 to 80% and more preferably 60 to 70%; the 3-isothiazolone comprises 0.5 to 15%, preferably 2 to 10% and more preferably 2 to 6%; and the surfactant comprises zero to 20%, preferably 1 to 15% and more preferably 2 to 10%, based on total weight of the composition.

The antimicrobial compositions of the present invention can be used to inhibit the growth of microorganisms by introducing a microbicidally effective amount of the composition onto, into, or at a locus subject to microbial attack. Suitable loci include, for example: cooling towers; air washers; boilers; mineral slurries; wastewater treatment; ornamental fountains; marine structures, such as boats, ships, oil platforms, piers, pilings, docks, elastomeric rubbers and fish nets; marine antifouling coatings, such as marine paints and varnishes; reverse osmosis filtration; ultrafiltration; ballast water; evaporative condensers; heat exchangers; pulp and paper processing fluids; plastics; emulsions and dispersions; paints; latexes; coatings, such as varnishes; construction products, such as mastics, caulks, and sealants; construction adhesives, such as ceramic adhesives, carpet backing adhesives, and laminating adhesives; industrial or consumer adhesives; photographic chemicals; printing fluids; household products, such as bathroom disinfectants or sanitizers; cosmetics and toiletries; shampoos; soaps; detergents; industrial disinfectants or sanitizers, such as cold sterilants, hard surface disinfectants; floor polishes; laundry rinse water; metalworking fluids; conveyor lubricants; hydraulic fluids; leather and leather products; textiles; textile products; wood and wood products, such as plywood, chipboard, flakeboard, laminated beams, oriented strandboard, hardboard, and particleboard; petroleum processing fluids; fuel; oilfield fluids, such as injection water, fracture fluids, and drilling muds; agriculture adjuvant preservation; surfactant preservation; medical devices; diagnostic reagent preservation; food preservation, such as plastic or paper food wrap; and pools and spas. The preferred end use applications of the compositions of the present invention are to protect cooling towers, air -washers, boilers, mineral slurries, wastewater treatment, ornamental fountains, reverse osmosis filtration, ultrafiltration, ballast water, evaporative condensers, heat exchangers, pulp and paper processing fluids, plastics, emulsions and dispersions, paints, latexes, coatings and metalworking fluids from-microorganisms.

The amounts of the compound to be used depend on the application. The useful amounts for a particular application are similar to amounts used for other antimicrobial compounds. Conventional methods for applying the compositions of -the present invention may be used, for example, dropping, intermittent adding, coating, spraying and dipping. In general, the final concentration of the antimicrobial compound in an aqueous system to be a locus is from 1 to 100 ppm, preferably from 10 to 50 ppm. For example, in the case of 4,5-dichloro-2-n-octyl-3-isothiazolone and 5-chloro-2-methyl-3-isothiazalone, the concentration is preferably 3 to 30 ppm, more preferably 3 to 10 ppm, and most preferably 3 to 6 ppm. In the case of a halocyanoacetamide, such as DBNPA, the concentration is preferably 10 to 100 ppm, more preferably 10 to 50 ppm, and most preferably 10 to 20 ppm.

Some embodiments of the invention are described in-detail in the following Examples; unless otherwise specified, all ratios, parts and percentages (%) are expressed by weight and all reagents used are of good commercial quality. Abbreviations used in the Examples and Tables are listed below:

| | |
|---|---|
| DBPNA | = 2,2-dibromo-3-nitrilopropionamide |
| DCOIT | = 4,5-dichloro-2-n-octyl-3-isothiazolone |
| CMIT | = 5-chloro-2-methyl-3-isothiazolone |
| MIT | 2-methyl-3-isothiazolone |
| GTA | = glyceryl triacetate (triacetin) |
| PGMEA | = propylene glycol methyl ether acetate |
| HPLC | = high pressure liquid chromatography |

### Example 1

To test the stability of DBNPA in various solvents, samples were prepared by combining 2.0 g DBNPA with 8.0 g solvent in 30 ml glass screw cap vials. Samples were capped and shaken until all of the DBNPA was dissolved, then stored at 60°C and analyzed by high pressure liquid chromatography (HPLC) with UV detection at the indicated time intervals. Results are shown in Table 1.

**Table 1**

| Solvent | % DBNPA retained | | | |
|---|---|---|---|---|
| | 1 week | 2 weeks | 3 weeks | 4 weeks |
| 86% dipropylene glycol in water* | 49 | NA | NA | NA |
| 75% dipropylene glycol in water* | 79 | NA | NA | NA |
| 62% dipropylene glycol in water* | 95 | NA | NA | 20 |
| dimethyl formamide* | 73 | 46 | NA | NA |
| triethylene glycol dimethyl ether* | 15 | 0 | NA | NA |
| octyl pyrrolidone* | 62 | 50 | 45 | NA |
| methyl amyl ketone* | 9 | 12 | 0 | NA |
| PGMEA* | 80 | 65 | 61 | 45 |
| ethyl acetate | 100 | 100 | 100 | 100 |
| GTA | 92 | 100 | 100 | 100 |
| diethyl phthalate | 100 | 98 | 98 | 98 |

| | | | | |
|---|---|---|---|---|
| * = comparative, not representative of the present invention. NA= not analyzed | | | | |

The above results demonstrate that halocyanoacetamides, such as DBNPA, are unstable (less than 50% retained after 4 weeks) in glycol, amide, ether and ketone solvents, but are exceptionally stable in the selected ester solvents.

### Example 2

Emulsifiable concentrate formulations containing DBNPA were prepared in 30 ml glass screw cap vials according to the amounts shown in Table 2 to test the stability of halocyanoacetamide in the presence of surfactant. Samples were prepared by mixing the ingredients in the vials which were then capped, shaken until the DBNPA dissolved.

The compositions containing DBNPA, GTA and surfactant (Tween™ 20 or Neodol™ 91-8 (C₉-C₁₁ linear primary alcohol ethoxylate)) and a comparative composition using 75% dipropylene glycol in water (with 0.4% butylated hydroxytoluene) as solvent, were stored at 55°C for 6 weeks and the concentration of active ingredient (DBNPA) in the compositions was determined by HPLC at the indicated time intervals. The results are shown in Table 2.

**Table 2**

| | Composition (grams) | | |
|---|---|---|---|
| Sample | DBPNA | GTA | Surfactant |
| Comparative* | 2.00 | ------ | -------- |
| 1 | 2.70 | 7.30 | 1.0 (Neodol™ 91-8) |
| 2 | 2.85 | 6.65 | 0.5 (Neodol™ 91-8) |
| 3 | 2.70 | 7.30 | 1.0 (Tween™ 20) |
| 4 | 2.85 | 6.65 | 0.5 (Tween™ 20) |

| | % DBNPA retained | | | | | |
|---|---|---|---|---|---|---|
| Sample | 1 Week | 2 Weeks | 3 Weeks | 4 Weeks | 5 Weeks | 6 Weeks |
| Comparative* | 87 | 78 | 58 | 33 | 0 | 0 |
| 1 | 93 | 93 | 87 | 82 | 78 | 74 |
| 2 | 96 | 96 | 92 | 89 | 88 | 84 |
| 3 | 97 | 94 | 89 | 86 | 82 | 79 |
| 4 | 97 | 96 | 92 | 91 | 90 | 88 |

| | | | | | | |
|---|---|---|---|---|---|---|
| * solvent = 8.0 g of 75% dipropylene glycol in water with 0.4% butylated hydroxytoluene | | | | | | |

These data demonstrate that emulsifiable concentrates of the invention are more stable than glycol solutions which were the state of the art prior to the present invention. All GTA/surfactant compositions of DBNPA retain at least 80% active ingredient after 4 weeks at 55°C.

### Example 3

Samples were prepared to test the stability of 5-chloro-2-methyl-3-isothiazolone (CMIT) and DBNPA mixtures. The compositions contained 25% DBNPA, 25% of mixture containing 19.75% CMIT and 6.19% 2-methyl-3-isothiazolone (MIT), zero or 5% surfactant, and 45 or 50% GTA. Surfactants were Tween™ 20, Triton™ DF-12 or Triton™ CF-10. The compositions were stored at 50°C for 4 weeks and the concentrations of active ingredients (DBNPA and CMIT) in the compositions were determined by HPLC at the indicated time intervals. The results are shown in Table 3.

**Table 3**

| % DBNPA retained | | | | | |
|---|---|---|---|---|---|
| Surfactant | 0 Time | 1 Week | 2 Weeks | 3 Weeks | 4 Weeks |
| Tween™ 20 | 100 | 97 | 71 | 61 | 54 |
| Triton™ DF-12 | 100 | 98 | 95 | 73 | 68 |
| Triton™ CF-10 | 100 | 97 | 93 | 77 | 69 |
| None | 100 | 98 | 90 | 85 | 78 |

| % CMIT retained | | | | | |
|---|---|---|---|---|---|
| Surfactant | 0 Time | 1 Week | 2 Weeks | 3 Weeks | 4 Weeks |
| Tween™ 20 | 100 | 100 | 80 | 75 | 74 |
| Triton™ DF-12 | 100 | 100 | 99 | 87 | 85 |
| Triton™ CF-10 | 100 | 98 | 96 | 86 | 84 |
| None | 100 | 102 | 97 | 90 | 88 |

These data demonstrate satisfactory stability of both CMIT and DBNPA in GTA solutions in the presence of surfactants: that is, greater than 50% active ingredient retained after 4 weeks at 50°C.

### Example 4

Samples were prepared to test the stability of 4,5-dichloro-2-n-octyl-3-isothiazolone (DCOIT) and DBNPA mixtures. An isothiazolone composition was prepared by mixing 16 g of DCOIT, 20 g of surfactant and 64 g of GTA. A DBNPA composition. was prepared by mixing 33.3 g of DBNPA and 66.6 g of GTA. The isothiazolone composition was mixed with the DBNPA composition at the ratio of 1:3. The final mixture contained 4% DCOIT, 25% DBNPA, 5% surfactant and 66% GTA. Surfactants were Tween™ 20, Triton™ DF-12 or Triton™ CF-10. These compositions were stored at 40°C for 6 weeks and the concentrations of active ingredients (DBNPA and DCOIT) in the compositions were determined by HPLC at the indicated time intervals. The results are shown in Table 4.

**Table 4**

| % DBNPA retained | | | |
|---|---|---|---|
| | Time | | |
| Surfactant | 0 | 4 Weeks | 6 Weeks |
| Tween™ 20 | 100.0 | 93.3 | 92.1 |
| Triton™ DF-12 | 100.0 | 92.3 | 92.0 |
| Triton™ CF-10 | 100.0 | 93.5 | 91.2 |

| % DCOIT retained | | | |
|---|---|---|---|
| | Time | | |
| Surfactant | 0 | 4 Weeks | 6 Weeks |
| Tween™ 20 | 100.0 | 94.3 | 94.0 |
| Triton™ DF-12 | 100.0 | 100.0 | 98.2 |
| Triton™ CF-10 | 100.0 | 96.0 | 95.6 |

The results demonstrate satisfactory stability of both DCOIT and DBNPA in GTA solutions in the presence of surfactants: that is, greater than 90% active ingredient retained after 6 weeks at 50°C.

### Example 5

Samples were prepared to test the freeze/thaw stability of compositions of the present invention. The freeze/thaw test was conducted for compositions of DCOIT, DBNPA, surfactant and GTA prepared in Example 4. Samples (20 g each) were stored at -10°C or -25°C for 19 hours to be frozen; samples were then stored at 25°C for 5 hours to be thawed. This sequence was repeated three times. Then the concentrations of active ingredients (DBNPA and DCOIT) in the compositions were determined by HPLC. The results are shown in Table 5.

**Table 5**

| % Active Ingredient Retained after Freezing and Thawing | | |
|---|---|---|
| Surfactant | % DBNPA retained Temperature | |
| | -10°C | -25°C |
| Tween™ 20 | 99.2 | 99.1 |
| Triton™ DF-12 | 98.8 | 99 |
| Triton™ CF-10 | 98.8 | 98.8 |

| Surfactant | % DCOIT retained Temperature | |
|---|---|---|
| | -10°C | -25°C |
| Tween™ 20 | 100.0 | 100.0 |
| Triton™ DF-12 | 100.0 | 100.0 |
| Triton™ CF-10 | 100.0 | 100.0 |

The results demonstrate satisfactory stability (clear solution, no precipitation or phase separation observed) of both DCOIT and DBNPA in GTA solutions in the presence of surfactants during freeze/thaw cycling: greater than 98% active ingredient retained.

### Example 6

This example illustrates the use of the emulsifiable concentrate as applied in aqueous systems by dilution. The dilution test was conducted for the compositions containing DCOIT, DBNPA, surfactant and GTA prepared in Example 4; the composition where surfactant is excluded from the composition has a final concentration 4% DCOIT, 25% DBNPA and 71% GTA. Compositions were diluted and made up to 20 g with distilled water in a 30 ml glass vial at the ratio of 1:30 to 1:10,000 (composition to water). Sample vials were capped and then inverted 15 times. Samples were allowed to stand at ambient conditions and the physical appearances of the mixtures were recorded. The results are shown in Table 6.

**Table 6**

| Appearance after Dilution | | | | |
|---|---|---|---|---|
| | Dilution Rate (GTA composition:water) | | | |
| Surfactant | 1:30 | 1:100 | 1:1,000 | 1:10,000 |
| None | S | S | S | C |
| Tween™ 20 | E | E | E | C |
| Triton™ DF-12 | S | E | E | C |
| Triton™ CF-10 | S | E | E | C |

| | | | | |
|---|---|---|---|---|
| C = dissolution (clear) | | | | |
| E = emulsion | | | | |
| S = phase separation | | | | |

The composition without surfactant undergoes phase separation at a ratio of 1:1,000, and does not form an emulsion. In the case of adding surfactant to the compositions, the compositions containing Triton™ DF-12 or Triton™ CF-10 underwent phase separation at the 1:30 ratio; however, these compositions formed an emulsion when the dilution rate was 1:100 or greater. The composition containing Tween™ 20 formed an emulsion at all dilution ratios. By adding surfactant, the emulsion forming capability of the compositions of the present invention is enhanced upon dilution into aqueous systems.

## Claims

1. A composition comprising:
(a) a halocyanoacetamide compound of formula **I**: 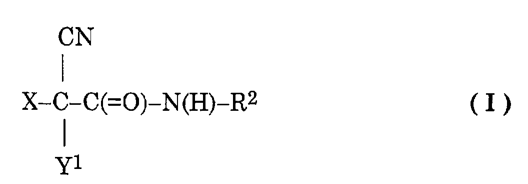 wherein:
X is hydrogen or halogen atom, Y¹ is halogen atom and R² is hydrogen or (C₁-C₆)alkyl group; and
(b) a solvent comprising a (C₁-C₄)alkyl ester of (C₁-C₃)aliphatic carboxylic acid or (C₇-C₉)aromatic carboxylic acid.

2. The composition of claim 1 wherein the halocyanoacetamide is selected from one or more of 2,2-dibromo-3-nitrilopropionamide, 2-chloro-3-nitrilopropionamide, 2-bromo-3-nitrilopropionamide, 2,2-dichloro-3-nitrilopropionamide and N-methyl-2,2-dibromo-3-nitrilopropionamide.

3. The composition of claim 1 wherein the solvent is selected from one or more of methyl formate, ethyl formate, propyl formate, butyl formate, methyl acetate, ethyl acetate, propyl acetate, butyl acetate, methyl propionate, ethyl propionate, propyl propionate, butyl propionate, glyceryl triacetate, glyceryl tripropionate, dimethyl phthalate, diethyl phthalate, dipropyl phthalate and dibutyl phthalate.

4. The composition of claim 1 further comprising a surfactant selected from one or more of non-ionic, cationic and anionic surfactant.

5. The composition of claim 4 wherein the surfactant is a non-ionic surfactant selected from one or more of ethoxylated (C₈-C₂₀)alcohols, ethoxylated sorbitan esters, ethoxylated (C₁₀-C₂₀)oils and fats, ethoxylated (C₁₀-C₂₀)fatty acids, ethoxylated (C₈-C₂₀)monoalkyl- or dialkyl-phenols, alkylaryl polyethers, ethoxylated alkanolamides and ethoxylated castor oil.

6. The composition of claim 1 further comprising a 3-isothiazolone compound of formula **II**: 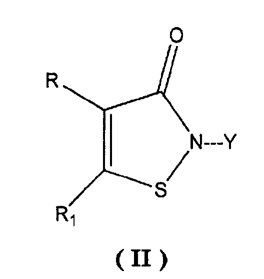 wherein:
Y is an unsubstituted or substituted (C₁-C₁₈)alkyl group, an unsubstituted or substituted (C₃-C₁₈)alkenyl or alkynyl group, an unsubstituted or substituted (C₅-C₁₂)cycloalkyl group, an unsubstituted or substituted (C₇-C₁₀)aralkyl group, or a substituted or unsubstituted (C₇-C₁₀)aryl group;
R and R₁ are independently hydrogen, halogen or (C₁-C₄)alkyl groups; or
R and R₁ can be taken together with the C=C double bond of the isothiazolone ring to form an unsubstituted or substituted benzene ring.

7. The composition of claim 6 further comprising a non-ionic surfactant selected from one or more of ethoxylated (C₈-C₂₀)alcohols, ethoxylated sorbitan esters, ethoxylated (C₁₀-C₂₀)oils and fats, ethoxylated (C₁₀-C₂₀)fatty acids, ethoxylated (C₈-C₂₀)monoalkyl- or dialkyl- phenols, alkylaryl polyethers, ethoxylated alkanolamides and ethoxylated castor oil.

8. The composition of claim 6 wherein the halocyanoacetamide is 2,2-dibromo-3-nitrilopropionamide, the solvent is glyceryl triacetate and the 3-isothiazolone is 4,5-dichloro-2-n-octyl-3-isothiazolone.

9. A method of stabilizing halocyanoacetamide compounds of formula **I**: 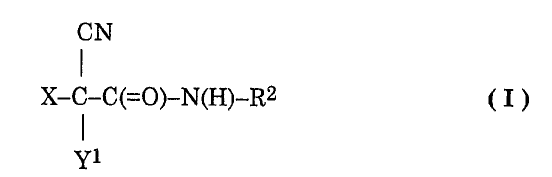 wherein:
X is hydrogen or halogen atom, Y¹ is halogen atom and R² is hydrogen or (C₁-C₆)alkyl group; comprising
(a) forming a solution by combining the halocyanoacetamide with an ester solvent comprising a (C₁-C₄)alkyl ester of (C₁-C₃)aliphatic carboxylic acid or (C₇-C₉)aromatic carboxylic acid; or
(b) forming an emulsifiable concentrate by combining the halocyanoacetamide with the ester solvent and one or more surfactants selected from non-ionic, cationic and anionic surfactant.
10. The method of claim 9 wherein the ester is selected from one or more of methyl formate, ethyl formate, propyl formate, butyl formate, methyl acetate, ethyl acetate, propyl acetate, butyl acetate, methyl propionate, ethyl propionate, propyl propionate, butyl propionate, glyceryl triacetate, glyceryl tripropionate, dimethyl phthalate, diethyl phthalate, dipropyl phthalate and dibutyl phthalate.
